# EUROPEAN PATENT SPECIFICATION

(11) **EP 2 732 874 B1**
(45) Date of publication and mention of the grant of the patent: **27.05.2015**
(21) Application number: 13193738.5
(22) Date of filing: 20.11.2013
(51) Int. Cl.: B01J 31/06, C07C 29/38, C07C 201/12

(54) **Method for synthesising heterogeneous solid chiral catalysts and their use in stereoselective reactions**
Verfahren zur Synthese von heterogenen, festen, chiralen Katalysatoren und deren Verwendung in stereoselektiven Reaktionen
Procédé de synthèse de catalyseurs chiraux solides hétérogènes et leur utilisation dans des réactions stéréosélectives

(30) Priority: 20.11.2012 MX 2012013420
(43) Date of publication of application: 21.05.2014
(73) Proprietor: Universidad de Guanajuato, C.P. 36000 Guanajuato (MX)
(72) Inventor: Galindo Esquivel, Ignacio René, 36000 Guanajuato (MX); Juárez Ruiz, Juan Manuel, 36000 Guanajuato (MX); Regalado Oliva, Orlando, 36000 Guanajuato (MX)
(74) Representative: Elzaburu Marquez, Alberto

(56) References cited:
- EP-A1- 1 479 439
- ES-A1- 2 349 604
- US-B2- 8 148 287

## Description

### OBJECT OF THE INVENTION

This invention describes a method for synthesis of heterogeneous solid chiral catalysts and their use in stereoselective reactions. Specifically, the invention describes a method for obtaining organic catalysts immobilised on a silicon oxide matrix. These catalysts can be applied in certain stereoselective reactions, are easily recovered from the reaction mixture, and can be reused.

### BACKGROUND

is a group of molecules known as chiral molecules that have identical chemical formulas, but which have different spatial distributions. These molecules possess very similar chemical and physical properties, which makes them difficult to separate using the methods typically employed in the chemical industry (crystallisation, absorption, etc.). On the other hand, unlike the differences in their physical and chemical properties, the biological properties of these molecules can be extremely different. For example, one molecule with a three-dimensional arrangement can have a harmful effect on a living organism, while another with the opposite arrangement can act as a medication. Phenomena like this are common and widely understood in the chemistry of enzymes, where the natural chemistry is stereoselective, or in other words, where it favours the formation of only one of the two three-dimensional arrangements that a chiral molecule can present. Each compound with a specific three-dimensional arrangement is known as an "enantiomer".

A large number of medications available on the market use chiral molecules as active ingredients. Since 1992, the FDA has banned the use of racemic mixtures (a 50/50 mixture of enantiomers) when one of the enantiomers has unknown or adverse pharmacological activity. Currently, the pharmaceutical industry is directing its efforts towards the ability to offer enantiomerically pure medications. However, medications with racemic mixtures are still being sold. When it is shown that one of the enantiomers has adverse effects on the organism, the mixture of enantiomers must be separated until only the desired active enantiomers is obtained.

As mentioned above, enantiomers present very similar physical and chemical properties and this makes them difficult to separate. The preferred procedure for production of pharmaceuticals involves the synthesis of racemic mixtures using non-selective catalysts. Once the racemic mixture is obtained, a series of separations are performed using diasteromeric resolution until the desired purity of the active enantiomer is achieved. To perform the separation a pure enantiomeric compound is used (generally obtained from some natural source), which is made to react with the racemic mixture in order to obtain a mixture of diasteromers. Unlike the enantiomers, the diasteromers have different physical properties, which allow them to be separated (generally by crystallisation). Once separated, the inverse of the reaction carried out to form the diasteromers is performed in order to obtain the desired enantiomer and recover the auxiliary chiral compound used in the separation, which can be recycled. This procedure generates low yields as well as useless sub-products.

An alternative process has been proposed where a stereoselective catalyst is used during synthesis of the chiral compound. This approach favours the formation of one of the enantiomers and decreases or eliminates the need for purification processes after the reaction has occurred.

For approximately the last decade, there has been an active search for stereoselective catalysts. The most widely researched chiral catalysts involve "organometalic" molecules that include precious metals such as Pt, Pd, Rh, etc. in their formulation. Catalysts of this type are generally sensitive to atmospheric oxidants, which causes greater difficulties in relation to handling during their synthesis and their use as catalysts in the production of chiral molecules. Additionally, catalysts of this type are homogeneous, which makes it necessary to perform a separation of the catalyst and the reaction mixture downstream. In order to overcome this last difficulty, the anchoring of organometallic catalysts to a variety of substrates has been proposed [US6028025, US5990318].

As an alternative to these organometallic catalysts, there are also chiral organic molecules that do not contain metals within their formulation, known as "organocatalysts" [US8084641, US7541456, US7291739]. One of their principal advantages is that they are not sensitive to atmospheric oxidants, although since they are less active than organometallic catalysts they require longer reaction times. These catalysts are also homogeneous, although to date there have been few efforts to immobilise systems of this type on solid matrices [US8148287, K. Sakthivel, W. Notz, T. Bui, C. F. Barbas III, J. Am. Chem. Soc. 123, 5260-5267, 2001; F. Calderón, R. Fernandéz, F. Sánchez, A. Fernández-Mayoralas, Adv. Synth. Catal. 347, 1395-1403, 2005; E.G. Doyagüez, F. Calderón, F. Sánchez, A. Fernández-Mayoralas, J. Org. Chem. 72, 9353-9356, 2007]. Patent US8148287 describes the anchoring of organic catalysts on a silicon foam using 1,2,3-triazole as the link between the inorganic and organic parts. F. Calderón *et al.* and E.G. Doyagüez *et al.* have developed a method for synthesising heterogeneous chiral catalysts using organic 4-hydroxyproline as a catalyst, which is anchored on previously synthesised oxide media: MCM-41, silica, ITQ2, and ITQ6. The aminoacid is anchored to the solid matrix through the hydroxyl group. In a similar manner, K. Sakthivel *et al.* have used a method of immobilising proline on a medium of silica gel, although the aminoacid is not anchored to the medium with a covalent bond. There is a recent report where proline anchored on mesostructured catalysts is synthesised in a single step [E.A. Prasetyanto, S.C. Lee, S.M. Jeong, S.E. Park, Chem. Commun. 1995-1997, 2008]. In this procedure, the aminoacid is anchored to the mesoporous support via the carboxyl group of the proline. In general, the processes used to immobilise organic catalysts involve anchoring of the organic catalyst through the formation of covalent bonds with a previously synthesised oxide surface, which allows for a maximum of approximately 10% by weight of the organic catalyst to be anchored. Very recently, in 2009 García-Doyagüez et al. filed patent ES2349604 in which they describe the construction of an organic hydroxyproline polymer and describe its use as a catalyst. This invention uses organic polymers based on vinyl monomers, and they report aldolic condensation activity with minimum reaction times of 24 hours and 97% conversion, obtaining enantiomeric selectivities with a 1:1 ratio for the most active catalyst.

The reaction times reported for achieving conversions of greater than 80% during aldolic condensation reactions vary between 24 and 72 hours, when homogeneous organic catalysts are used [K. Sakthivel, W. Notz, T. Bui, C. F. Barbas III, J. Am. Chem. Soc. 123, 5260-5267, 2001], and between approximately 24 and 48 hours when heterogeneous catalysts obtained by the traditional anchoring methods available in the literature are used. [F. Calderón, R. Fernández, F. Sánchez, A. Fernández-Mayoralas, Adv. Synth. Catal. 347, 1395-1403, 2005; E.G. Doyagüez, F. Calderón, F. Sánchez, A. Fernández-Mayoralas, J. Org. Chem. 72, 9353-9356, 2007]. The reaction times observed when using the catalysts obtained by following the method described in the present invention vary between 2 and 6 hours.

The present invention reports on a new and innovative method for synthesis of heterogeneous chiral catalysts, which consists of generating the solid silicon oxide matrix using a monomer functionalised using the duly protected organic catalyst, and which allows heterogeneous solid chiral catalysts with approximately 30% organic catalyst by weight to be obtained. The process for this synthesis includes four economically accessible reaction steps that are simple to perform. These are high yield and do not require sophisticated purification, and only in steps three and four does the solid product require simple washing with an organic or aqueous solvent. Additionally, the catalysts obtained with the present invention catalyse condensation reactions in a stereoselective manner.

### DETAILED DESCRIPTION OF THE INVENTION

The proposed invention refers to the development of a method for obtaining heterogeneous solid chiral catalysts that consist of one part α-aminoacid and another inorganic part that consists of a silicon oxide matrix; as well as its application in reactions where stereoselective carbon-carbon bonds are formed.

The method for synthesising the catalyst can be applied to α-aminoacids such as: valine, proline, alanine, phenylglycine, phenylalanine, leucine, isoleucine, and methionine.

A method for synthesising heterogeneous solid chiral catalysts where the method consists of the following 4 reaction steps described here:

### Step I Reaction for protection of the amino group of the α-aminoacid.

The first step consists of a reaction for protection of the amino group of the α-aminoacid using *tert*-butyloxycarbonyl (Boc).

First, 0.5 to 3.0 equivalents of di-tert-butyl dicarbonate (Boc₂O) are added to a 0.1 to 2.0M solution of α-aminoacid in a mixture of distilled water and 1,4-dioxane (in a ratio of 1:0.5 to 1:2 by volume) at 0 °C to 30 °C. Next, 0.3 to 3.0 equivalents of triethylamine (TEA) are added to the reaction mixture. The reaction mixture is stirred continuously for 12 to 72 hours at 20 °C to 40 °C. The reaction mixture is partially concentrated and the solution is extracted with ethyl acetate. The organic phase is separated and dried using anhydrous sodium sulphate or magnesium sulphate. The solution is then concentrated to obtain the raw product.

### Step II Silanisation reaction between α-aminoacid protected with tert-butyloxycarbonyl (Boc), product of Step I and aminopropyltriethoxysilane (APTES).

A 0.1 to 2.0 solution of α-aminoacid protected with Boc in acetonitrile (CH₃CN) or methanol (CH₃OH) stirred at 0°C to 40°C under nitrogen fixation is held at 0.5 to 3.0 equivalents of potassium carbonate (K₂CO₃) and from 0.5 to 3.0 equivalents of *O-*(benzotriazole-1-il)-*N,N,N',N'*-tetramethyluronio-tetrafluoroborate (TBTU). The reaction mixture is continuously stirred from 10 to 60 minutes. Add 0.5 to 5.0 equivalents of APTES to the reaction mixture. The reaction mixture is maintained at 20°C to 40°C and from 12 to 72 hours. The reaction mixture is filtered and the filter is washed with acetonitrile or methanol. The filter is vacuum concentrated to obtain the raw product that corresponds with the α-aminoacid protected with Boc and bonded by the carboxile group with the APTES aminoacid group.

### Step III Inorganic polymerisation reaction of the raw product in Step II.

A 0.1 to 2.0M solution of raw compound obtained in Step II in ethanol (CH₃CH₂OH) or methanol is added to 1.0 to 20 equivalents of silicon alkoxide. Additionally, 0.1 to 3.0 equivalents of a basic catalyst (such as potassium carbonate) or an acid catalyst (such as acetic acid (CH₃CO₂H)) is added to this mixture. The reaction mixture is heated at 40°C to 80°C for 0.1 to 12 hours. 30 to 100 equivalents of distilled water is slowly added to the reaction mixture with a temperature of 40 to 80°C. The reaction mixture is continuously stirred at 40 to 80°C for 0.5 to 24 hours (until gel formation). The reaction mixture is set to rest at 20 to 30°C with ventilation for 24 to 72 hours. The gel dries at 40°C to 90°C and from 24 to 72 hours. The solid is pulverised and washed with methanol and dichloromethane (CH₂Cl₂) to obtain the solid heterogeneous chiral catalyst protected with Boc.

### Step IV Deprotection or activation of the solid heterogeneous chiral catalyst during Step III.

In a suspension from the Step III product (or Step VI) in dichloromethane, chloroform (CHCl₃) or dichlorethane (ClCH₂CH₂Cl) in mass/volume proportion (g/mL) of 1/10, 3M hydrochloric acid or trifluoroacetic acid (CF₃CO₂H) in ethyl acetate (HCl/EtOAc) in proportion to mass/volume (g/mL) of 1/0.1 to 1/5 is added. The reaction mixture of 0 to 25°C is stirred for 3 to 72 hours. The reaction mixture is filtered and the solid is neutralized as an aqueous solution of Na₂CO₃ or NaHCO₃ to 10 or 20% The activated solid heterogeneous chiral catalyst is filtered and washed with distilled water. The catalyst dries at 40°C to 80°C and from 10 to 72 hours.

When the described method of this invention works, it is necessary to perform each and every one of Steps I, II, III, and IV mentioned. However, the fundamental step of the method is indicated in Step III, where inorganic polymerisation of silicon alkoxide is performed with the α - aminoacid protected with Boc and silanised (product of Step II). Alternatively, a method can be used to synthesise heterogeneous chiral catalysts, where α-aminoacid protected with Boc (product of Step II) is covalently bonded with an amino group from a solid matrix of silicon dioxide, described as follows:

### Step V Inorganic polymerisation of APTES.

A 0.1 to 2.0M solution of APTES in ethanol or methanol is added to 0.1 to 3.0 equivalents of potassium carbonate and 1.0 to 20 equivalents of silicon alkoxide. The reaction mixture is heated at 40°C to 80°C for 0.1 to 12 hours. 30 to 100 equivalents of distilled water is slowly added to the reaction mixture with a temperature of 40 to 80°C. The reaction mixture is continuously stirred at 40 to 80°C for 0.5 to 24 hours until gel formation. The reaction mixture is set to rest at 20 to 30°C with ventilation for 24 to 72 hours. The gel dries at 40°C to 90°C and from 24 to 72 hours. The solid is pulverized to obtain a silicone oxide powder which is highly substituted with surface amino groups.

### Step VI Reaction of solid matrix (product of Step V) with the α-aminoacid protected with Boc (product of Step I).

A 0.1 to 1.5M solution of α-aminoacid protected with Boc in acetonitrile or methanol stirred at 0°C to 40°C under nitrogen fixation is held at 0.5 to 3.0 equivalents of K₂CO₃ and from 0.5 to 3.0 equivalents of (TBTU). The reaction mixture is continuously stirred from 10 to 60 minutes. A solid obtained in Step V with a mass/volume proportion (g/mL) of 1/5 to 1/30 is added to the reaction mixture. The reaction mixture is maintained at 20°C to 40°C from 12 to 72 hours. The reaction mixture is filtered and the filter is washed with acetonitrile or methanol. The filter is vacuum dried to obtain the raw product.

The deprotection or activation reaction of the of the heterogeneous chiral catalyst obtained in Step IV is deprotected as described in Step IV.

### APPLICATION

The solid heterogeneous chiral catalyst obtained through any of these methods may be used in reactions to form carbon-carbon bonds of chiral nature, such as aldol condensation, Mannich condensation, Michael addition, Henry reaction using aqueous reaction methods or organic methods. The reactions can be performed from -78°C to 40°C.

### EXAMPLES

### EXAMPLE 1

Synthesising a solid heterogeneous chiral catalyst with a L-proline base.

### Step I

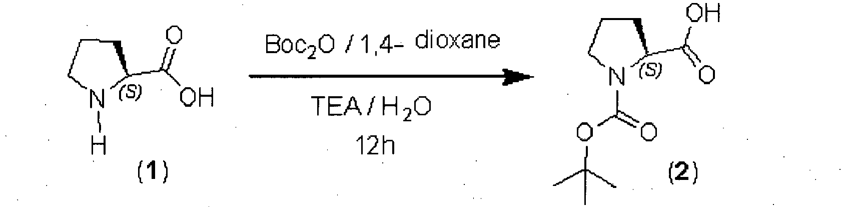

### Protection reaction with Boc from the L-proline (1) amino group

1.1 equivalents of Boc₂O are added to 0.5M L-proline solution (**1**) in a distilled water mixture and 1.4-dioxane (1:1) at a temperature of 0°C. Once the mixture is homogenised, 1.1 equivalents of TEA is slowly added. The reaction mixture is continuously stirred for 12 hours at 25°C. The reaction mixture is partially concentrated and the solution is extracted with ethyl acetate. The organic phase is separated and dried with anhydrous sodium sulphate. The solution is concentrated to obtain *N*-Boc L-proline (**2**) with a 98% yield. The product is not purified or subjected to the following reaction in its raw form.

### Step II

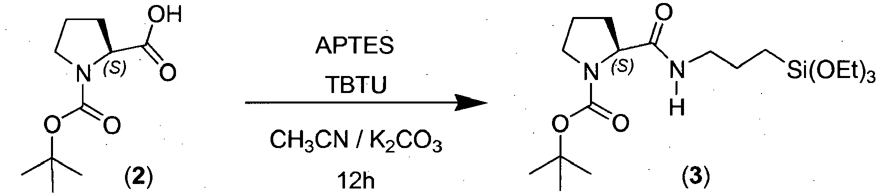

### Silanisation reaction of the compound (2) with APTES

1.0 equivalents of potassium carbonate and 1.5 equivalents of TBTU are added to a 0.2M solution of the compound (**2**) in acetonitrile at 0°C under nitrogen fixation. The mixture is continuously stirred for 60 minutes to later add 1.0 equivalent of APTES. The reaction mixture is maintained for 12 hours at 25°C. The reaction mixture is concentrated and ethyl acetate is added to the resulting viscose product and the suspension is filtered. The resulting solution is concentrated obtaining a slightly yellow viscose liquid (**3**).

### Step III

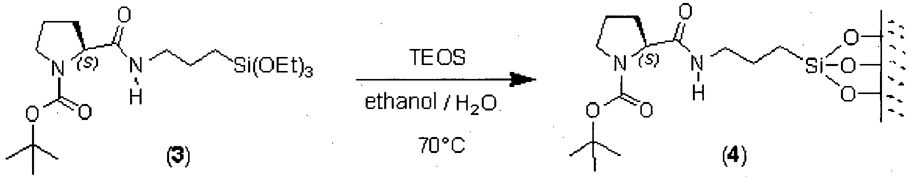

### Polymerisation reaction of the compound (3)

0.5 equivalents of potassium carbonate and 3.0 equivalents of tetraethylortosilicate (TEOS) are added to a 0.3M of compound (**3**) in ethanol. The reaction mixture is heated at 70°C for 2 hours. 30 equivalents of distilled water is slowly added to the reaction mixture with a temperature of 70°C. The reaction mixture is continuously stirred at 70°C until gel formation. The reaction mixture is set to rest at 25°C with ventilation for 24 to 72 hours. The gel dries at 60°C for 48 hours. The solid is pulverised and washed with methanol and dichloromethane to obtain a yellowish powder corresponding to the compound **(4).**

### Step IV

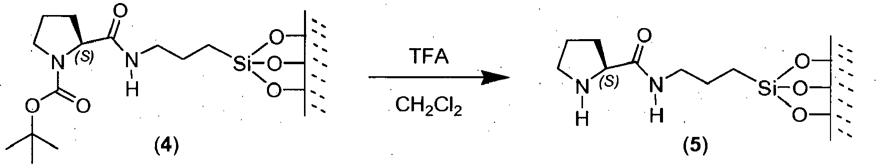

### Deprotection or activation reaction of the compound (4)

0.1 mL of trifluoroacetic acid is added to a suspension of the compound (**4**) in dichloromethane using 1g of solid for 10mL of solvent. The reaction mixture is maintained at 0°C and stirred for 3 hours. The reaction mixture is filtered and the solid is neutralised as an aqueous solution of Na₂CO₃ at 10%. The activated solid heterogeneous chiral catalyst is filtered and washed with distilled water. The catalyst dries at 80°C for 48 hours, obtaining a fine powder with a slightly yellow colour. If preferred, the catalyst (**5**) can be used in aqueous reactions without being previously dried.

The solid or compound heterogeneous chiral catalyst (**5**) is characterised through nuclear magnetic resonance of ¹³C of solids where signs corresponding to the expected catalyst are observed: ¹³C CP MAS NMR (16 kHz, CDCl₃, ppm): δ= 9.7 (C_{A}), 24 (C_{B}), 30.7 (C_{C}), 41.1 (C_{D}), 46.5 (C_{E}), 60.9 (C_{F}), 174.8 (C_{G}). The elemental analysis indicates the presence of 31.5% in organic catalyst weight in the heterogeneous chiral catalyst.

### EXAMPLE 2

### Synthesising a solid catalyst with a L-proline base.

### Step 1 was carried out as described in Example 1 to obtain N-Boc L-proline (2). Previously, esterification of N-Boc L-proline (2) with 9-bromononanol was completed to obtain the compound (6). The reaction proceeds in the following manner:

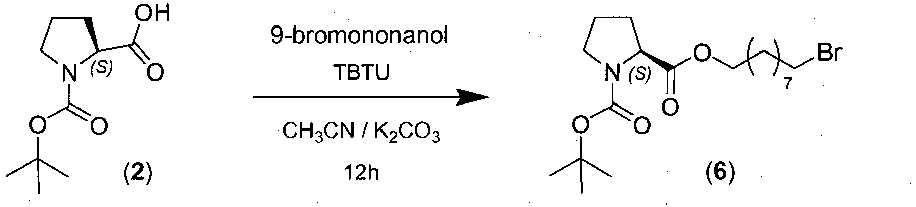

### Esterification of N-Boc L-proline

1.0 equivalents of potassium carbonate and 1.5 equivalents of TBTU are added to a 0.2M solution of *N*-Boc (**2**) in acetonitrile at 0°C under nitrogen fixation. The reaction mixture agitated for 60 minutes. 1.0 equivalents of 9-bromononanol is added to the reaction mixture. The reaction mixture is continuously stirred for 12 hours. The reaction mixture is concentrated and the product is purified for a chromatographic column using silica gel as the immobile phase and a mixture of ethyl hexanol/acetate (3/1) as mobile phase to obtain the compound (**6**) at a 92% yield.

### Step II is applied to compound (6) as follows:

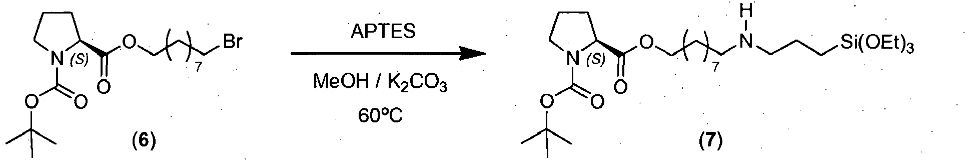

### Silanisation reaction of the compound (6) with APTES

1.0 equivalents of potassium carbonate and 1.0 equivalents of APTES are added to a 0.2M solution of the compound (**6**) in methanol at room temperature under nitrogen fixation. The reaction mixture is heated at 60°C for 12 hours. The reaction mixture is concentrated and the compound (**8**) without purification is used in the following reaction.

### Step III of inorganic polymerisation from Example 1 is applied to compound (7) but using 6.0 equivalents of TEOS.

### Step III is applied to compound (7) as follows:

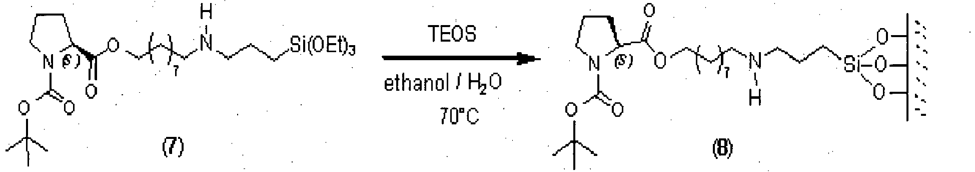

### Polymerisation reaction of the compound (7)

0.5 equivalents of potassium carbonate and 6.0 equivalents of TEOS are added to a 0.3M of compound (**7**) in ethanol. The reaction mixture is heated at 70°C for 2 hours. 30 equivalents of distilled water is slowly added to the reaction mixture with a temperature of 70°C. The reaction mixture is continuously stirred at 70°C until gel formation. The reaction mixture is set to rest at 25°C with ventilation for 24 to 72 hours. The gel dries at 60°C for 48 hours. The solid is pulverised and washed with methanol and dichloromethane to obtain a yellowish powder corresponding to the compound (**8**)**.**

### Step IV is applied to compound (8) as follows:

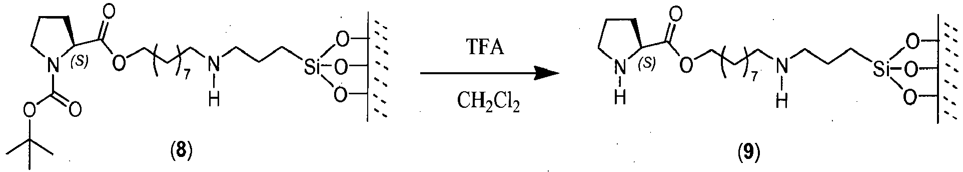

### Deprotection or activation reaction of the compound (8)

0.1 mL of trifluoroacetic acid is added to a suspension of the compound (**8**) in dichloromethane using 1.0g of solid for 10mL of solvent. The reaction mixture is maintained at 0°C and stirred for 3 hours. The reaction mixture is filtered and the solid is neutralised as an aqueous solution of 10% Na₂CO₃. The activated solid heterogeneous chiral catalyst is filtered and washed with distilled water. The catalyst dries at 80°C for 48 hours, obtaining a fine powder with a slightly yellow colour. If preferred, the catalyst (**9**) can be used in aqueous reactions without being previously dried.

The catalyst (**9**) is characterised through nuclear magnetic resonance of ¹³C of solids where signs corresponding to the expected catalyst are observed: ¹³CP MAS NMR (16 kHz, CDCl₃, ppm): δ= 9.8 (Cₐ), 20.5 (C_{b}), 28.7 (C_{c}), 43.9 (C_{d}), 49.1 (Cₑ), 51.8 (C_{f}), 59.7 (C_{g}), 67.5 (Cₕ), 162 (Cᵢ). The elemental analysis indicates the presence of 21% in organic catalyst in the final solid.

This example shows that Steps I, II, III, and IV, described in this invention, are always present to carry out the synthesis of solid catalysts with modified α-aminoacids, even though the modification of the α-aminoacids involves additional reaction steps. In order to obtain the solid heterogeneous chiral catalyst, each and every one of Steps I, II, III, and IV have been included, or Steps I, V, VI and IV described in the invention method have also been carried out.

### EXAMPLE 3

Use of catalyst (**5**) in the aldol condensation of 4-nitrobenzaldehyde with acetone.

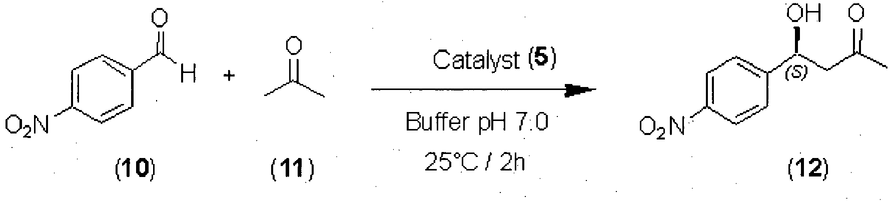

### Aldol condensation catalysed by means of catalyst (5)

To a 0.3M solution of 4-nitrobenzaldehyde (**10,** 0.661 mmol) in a pH=7 (0.05M) phosphate buffer at 25°C is added acetone (**11,** 3.305 mmol) and 0.1g of catalyst (**5**). The reaction mixture is continually stirred at 25°C for two hours. The suspension is filtered, the solution is partially concentrated and extracted using ethyl acetate. The organic phase is dried using anhydrous sodium sulphate and concentrated in a vacuum. The product is purified by means of a chromatography column to obtain the compound β-hydroxycetone (**12**) with 98% yield and 79% excess enantiomer, corresponding to (*S*) enantiomer.

### EXAMPLE 4

Use of catalyst (**5**) in the aldol condensation reaction of 3-nitrobenzaldehyde with acetone. In this reaction with 3-nitrobenzaldehyde the same reaction conditions used in Example 3 are employed in 3h. The corresponding β-hydroxycetone product is obtained with 92% yield and 64% excess (*S*) entantiomer.

### EXAMPLE 5

Use of catalyst (**5**) in the aldol condensation reaction of isatin with acetone.

In this reaction with isatin the same reaction conditions used in Example 3 are employed in 3h. The corresponding β-hydroxycetone product is obtained with 76% yield and 84% excess (*R*) entantomer.

### EXAMPLE 6

Use of catalyst (**9**) in the aldol condensation reaction of 4-nitrobenzaldehyde with acetone. In this reaction with catalyst (**9**) the same reaction conditions used in Example 3 are employed in 4h. The corresponding β-hydroxycetone product is obtained with 75% yield and 69% excess (*S*) enantiomer.

### EXAMPLE 7

Use of catalyst (**9**) in the aldol condensation reaction of 3-nitrobenzaldehyde with acetone.

In this reaction with catalyst (**9**) and 3-nitrobenzaldehyde the same reaction conditions used in Example 3 are employed in 4h. The corresponding β-hydroxycetone product is obtained with 67% yield and 55% excess (*S*) enantiomer.

### EXAMPLE 8

Use of catalyst (**9**) in the aldol condensation reaction of isatin with acetone.

In this reaction with catalyst (**9**) and isatin the same reaction conditions used in Example 3 are employed in 6 h. The corresponding β-hydroxycetone product is obtained with 58% yield and 76% excess (R) enantiomer.

### EXAMPLE 9

Reuse of catalyst (**5**) in the aldol condensation reaction of 4-nitrobenzaldehyde with acetone.

The aldol condensation reaction of 4-nitrobenzaldehyde with acetone is carried out using catalyst (**5**) as described in Example 3. Catalyst (**5**), which is filtered, is washed with acetone and added to a new 0.3M solution of 4-nitrobenzaldehyde (0.661 mmol) in a pH=7 (0.05M) phosphate buffer at 25°C with acetone (3.305 mmol). This reuse process is reproduced in several cycles, obtaining the results displayed in Table 1.

**Table 1. Reuse of catalyst (5) with 4-nitrobenzaldehyde and acetone**

| 4-nitrobenzaldehyde | Results | | |
|---|---|---|---|
| Reuse | Yield | time | Excess enantiomer (%) |
| 0 | 98% | 2h | 79(*S*)-(-) |
| 1 | 96% | 2h | 79 (*S*)-(-) |
| 2 | 92% | >2h | 79 (*S*)-(-) |
| 3 | 82% | >2h | 76 (*S*)-(-) |
| 4 | 80% | ≈3h | 69 (*S*)-(-) |

### EXAMPLE 10

Reuse of catalyst (**o**) in the aldol condensation reaction of 4-nitrobenzaldehyde with acetone.

The aldol condensation reaction of 4-nitrobenzaldehyde with acetone is carried out using catalyst (**9**) as described in Example 3. Catalyst (**9**), which is filtered, is washed with acetone and added to a new 0.3M solution of 4-nitrobenzaldehyde (0.661 mmol) in a pH=7 (0.05M) phosphate buffer at 25°C with acetone (3.305 mmol). This reuse process is reproduced in several cycles, obtaining the results displayed in Table I

**Table 2. Reuse of catalyst (9) with 4-nitrobenzaldehyde and acetone**

| 4-nitrobenzaldehyde | Results | | |
|---|---|---|---|
| Reuse | Yield | time | Excess enantiomer (%) |
| 0 | 75% | 4h | 69 (*S*)-(-) |
| 1 | 70% | 4h | 66 (*S*)-(-) |
| 2 | 70% | 5h | 63 (*S*)-(-) |
| 3 | 66% | 6h | 59 (*S*)-(-) |

## Claims

1. A compound of formula:
--- [O-Si-C₃H₆-NH-R₁-OCO-(CNH)-R₂-R₂']_{X}-[O-Si]_{Y}--- Formula I
wherein R1 is an aliphatic chain -(CH₂)ₙ- with n ranging between 0 and 9 and -OCO-(CNH)-R₂-R₂' corresponds to the α-aminoacid used as the organic catalyst; wherein said α-aminoacid is any of the ones specified in the following table:
| Name of the α-aminoacid | Chemical formula |
|---|---|
| Pro line | 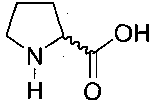 |
| Valine | 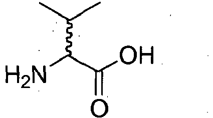 |
| Alanine | 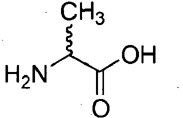 |
| Phenylglycine | 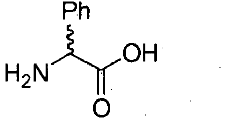 |
| Phenylalanine | 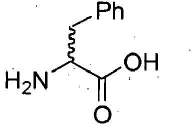 |
| Leucine | 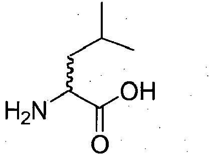 |
| Isoleucine | 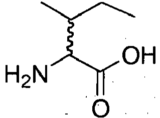 |
| Methionine | 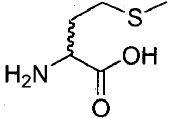 |
and wherein the values X and Y are maintained in a ratio ranging between 1:1 and 1:20.

2. The compound of Formula I as per claim 1, with n=0 and -OCO-(CNH)-R₂-R₂' corresponding to Proline.

3. The compound of Formula I as per claim 1, with n=9 and -OCO-(CNH)-R₂-R₂' corresponding to Proline.

4. The compound of Formula I as per claim 2 with values X=1 and Y= 3.

5. The compound of Formula I as per claim 3 with values X=1 and Y= 6.

6. A method to obtain the Compound of Formula I **characterised in that** it encompasses the following steps: protection of the amino group of an α-aminoacid by means of the reaction with a protective group such as Boc₂O (STEP I); silanisation of the protected α-aminoacid linking through the carboxyl group by means of aminopropyl triethoxysilane (STEP II); inorganic polymerisation of the protected and silanised aminoacid by means of a silicon alkoxide in the presence or absence of an acid or base catalyst, maintaining a protected and silanised aminoacid:alcoxide ratio that ranges between 1:1 and 1:20 (STEP III); removal of protection of the amino group of the α-aminoacid using 3M hydrochloric acid or trifluoroacetic acid in ethyl acetate (STEP IV).

7. A method to obtain the Compound of Formula I as per claim 6 **characterised in that** Step I is carried out using a solution of 0.1 to 2.0M α-aminoacid in a mix of distilled water and 1,4-dioxane (in a volume ratio ranging between 1:0.5 to 1:2) between 0°C and 30°C, adding between 0.5 to 3.0 equivalents of diterbutyl carbonate.

8. A method to obtain the Compound of Formula I as per claim 7, **characterised in that** between 0.3 and 3.0 equivalents of triethylamine are added and the mix is left to react between 20°C and 40°C for a period ranging between 12 and 72 hours.

9. A method to obtain the Compound of Formula I as per claim 6 **characterised in that** Step II is carried out using a mix of protected α-aminoacid in 0.1 to 2.0M acetonitrile or methanol, to which are added between 0.5 to 3.0 equivalents of potassium carbonate and between 0.5 to 3.0 equivalents of *O*-(benzotriazole-1-yl)-*N,N,N',N*'-tetrametiluronium-tetrafluoroborate, at a temperature ranging between 0°C and 40°C in a nitrogen atmosphere for a period of 10 to 60 minutes, followed by addition to this mix of between 0.5 to 5.0 equivalents of aminopropyltriethoxysilane, learning the mixture to react for a period ranging between 12 and 72 hours at a temperature between 20°C and 40°C.

10. A method to obtain the Compound of Formula I as per claim 9 **characterised in that** the product obtained in Step II is purified and washed using a suitable solvent such as acetonitrile or methanol.

11. A method to obtain the Compound of Formula I as per claim 6 **characterised in that** Step III is carried out using a solution of 0.1 to 2.0M of the protected and silanised α-aminoacid dissolved in ethanol or methanol at a temperature between 40 and 80°C and combining with potassium carbonate (or acetic acid) in a ratio of 0.1 to 3.0 equivalents for a period of 0.1 to 12 hours, whereinafter to this mix are slowly added between 1.0 to 20 equivalents of silicon alkoxide, and later between 30 to 100 equivalents of distilled water, continuously stirring the reaction mixture at 40 to 80°C for 0.5 to 24 hours.

12. A method to obtain the Compound of Formula I as per claim 11 **characterised in that**, in order to conclude Step III, the product is left to age and rest at a temperature of between 20 and 30°C for a period of between 24 and 72 hours, whereinafter, the material is dried at a temperature of 40 to 90°C for between 24 and 72 hours until a dry solid is obtained.

13. A method to obtain the Compound of Formula I as per claim 11 **characterised in that** at the end of Step III the product is purified and washed with methanol and dichloromethane.

14. A method to obtain the Compound of Formula I as per claim 6 **characterised in that** Step IV is carried out using 3M hydrochloric acid or trifluoroacetic acid in ethyl acetate in a ratio of 0.1 to 5.0 mL of acid for every 1g of the dry solid obtained in Step III.

15. A method to obtain the Compound of Formula I **characterised in that** it comprises the following steps : protection of the amino group of an α-aminoacid by means of the reaction with a protective group such as Boc₂O (STEP I); inorganic polymerisation of APTES with silicon alkoxide (STEP V); a fixation reaction between the protected α-aminoacid (the product of Step I) and the solid obtained in Step V (STEP VI); removal of the protection of the amino group of the α-aminoacid by means of 3M hydrochloric acid or trifluoroacetic acid in ethyl acetate (STEP IV).

16. A method to obtain the Compound of Formula I as per claim 15 **characterised in that** Step V is carried out using a mix of APTES dissolved in ethanol or methanol of between 0.1 and 2.0M at a temperature of 40 to 80°C, whereinafter to this mix are slowly added 1.0 to 20 equivalents of silicon alcoxide and later 30 to 100 equivalents of distilled water, continuously stirring the reaction mixture at 40 to 80°C for between 0.1 and 12 hours.

17. A method to obtain the Compound of Formula I as per claim 16 **characterised in that**, in order to end Step V, the product is left to age and rest at a temperature of between 20 and 30°C for a period of between 24 and 72 hours, whereinafter, the material is dried at a temperature of 40 to 90°C for between 24 and 72 hours until a dry solid is obtained.

18. A method to obtain the Compound of Formula I as per claim 15 **characterised in that** Step VI is carried out using a solution of between 0.1 to 1.5M of the α-aminoacid protected by Boc in acetonitrile or methanol stirred at between 0°C and 40°C in a nitrogen atmosphere, to which are added between 0.5 and 3.0 equivalents of potassium carbonate and between 0.5 and 3.0 equivalents of *O*-(benzotriazole-1-yl)-*N,N,N',N'-*tetrametiluronium-tetrafluoroborate, under continuous stirring for a time of between 10 and 60 minutes, whereinafter to the reaction mixture is added the solid obtained in Step V in a mass/volume ratio (g/mL) ranging between 1/5 and 1/30, subsequently maintaining the reaction mixture at 20°C to 40°C for between 12 and 72 hours.

19. A method to obtain the Compound of Formula I as per claim 18 **characterised in that** the product obtained in Step IV is purified and washed using a suitable solvent such as acetonitrile or methanol.

20. Use of the Compound of Formula I as per any of claims 1 to 5 as a catalyst in reactions of formation of carbon-carbon links of a chiral nature, such as aldolic condensation, Michael addition, Mannich reaction, or Henry reaction.

21. Use as per claim 20 using aqueous or organic means of reaction within the range of temperatures between -78°C to 40°C.

22. Use as per claim 20 using aqueous means of reaction with a base of pH 7 phosphate buffer within a range of temperatures between 0°C and 10°C.

## Patentansprüche

1. Verbindung der Formel:
--- [O-Si-C₃H₆-NH-R₁-OCO-(CNH)-R₂-R₂']_{X}-[O-Si]_{Y}--- Formel I
worin R1 eine aliphatische Kette -(CH₂)ₙ- ist, in der n im Bereich von 0 bis 9 liegt, und -OCO-(CNH)-R₂-R₂' der als organischer Katalysator eingesetzten α-Aminosäure entspricht; wobei die besagte α-Aminosäure eine der in der folgenden Tabelle aufgeführten Aminosäuren ist:
| Bezeichnung der α-Aminosäure | Chemische Formel |
|---|---|
| Prolin | 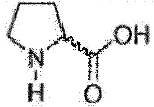 |
| Valin | 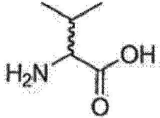 |
| Alanin | 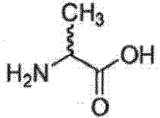 |
| Phenylglycin | 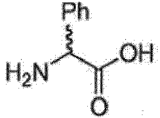 |
| Phenylalanin | 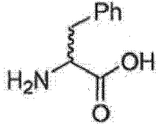 |
| Leucin | 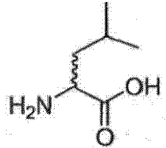 |
| Isoleucin | 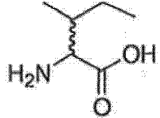 |
| Methionin | 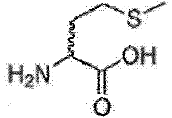 |
und wobei die Werte X und Y in einem Verhältnis von 1:1 bis 1:20 gehalten werden.

2. Verbindung der Formel I nach Anspruch 1, wobei n = 0 und -OCO-(CNH)-R₂-R₂' für Prolin steht.

3. Verbindung der Formel I nach Anspruch 1, wobei n = 9 und -OCO-(CNH)-R₂-R₂' für Prolin steht.

4. Verbindung der Formel I nach Anspruch 2, mit den Werten X = 1 und Y = 3.

5. Verbindung der Formel I nach Anspruch 3, mit den Werten X = 1 und Y = 6.

6. Verfahren zur Gewinnung der Verbindung der Formel I, **dadurch gekennzeichnet, dass** es folgende Schritte umfasst: Schützen der Aminogruppe einer α-Aminosäure durch Reaktion mit einer Schutzgruppe wie etwa Boc₂O (SCHRITT I); Silanisierung der geschützten α-Aminosäure über die Carboxylgruppe mittels Aminopropyltriethoxysilan (SCHRITT II); anorganische Polymerisierung der geschützten und silanisierten Aminosäure mithilfe eines Siliciumalkoxids in An- oder Abwesenheit eines sauren oder basischen Katalysators, wobei ein Verhältnis zwischen geschützter und silanisierter Aminosäure und Alkoxid im Bereich von 1:1 bis 1:20 eingehalten wird (SCHRITT III); Entfernen der Schutzgruppe von der Aminogruppe der α-Aminosäure unter Verwendung von 3 M Salzsäure oder Trifluoressigsäure in Ethylacetat (SCHRITT IV).

7. Verfahren zur Gewinnung der Verbindung der Formel I nach Anspruch 6, **dadurch gekennzeichnet, dass** Schritt I unter Verwendung einer 0,1- bis 2,0-molaren Lösung der α-Aminosäure in einer Mischung aus destilliertem Wasser und 1,4-Dioxan (in einem Volumenverhältnis im Bereich von 1:0,5 bis 1:2) bei 0°C bis 30°C und unter Zugabe von 0,5 bis 3,0 Äquivalenten Diterbutylcarbonat durchgeführt wird.

8. Verfahren zur Gewinnung der Verbindung der Formel I nach Anspruch 7, **dadurch gekennzeichnet, dass** zwischen 0,3 und 3,0 Äquivalente Triethylamin zugegeben werden und die Mischung zwischen 20°C und 40°C über einen Zeitraum von 12 bis 72 Stunden zur Reaktion gebracht wird.

9. Verfahren zur Gewinnung der Verbindung der Formel I nach Anspruch 6, **dadurch gekennzeichnet, dass** Schritt II unter Verwendung einer Mischung aus der geschützten α-Aminosäure in 0,1 bis 2,0 M Acetonitril oder Methanol durchgeführt wird, der 0,5 bis 3,0 Äquivalente Kaliumcarbonat und 0,5 bis 3,0 Äquivalente O-(Benzotriazol-1-yl)-N,N,N',N'-tetramethyluronium-tetrafluorborat bei einer Temperatur im Bereich von 0°C bis 40°C unter einer Stickstoffatmosphäre über einen Zeitraum von 10 bis 60 Minuten zugesetzt werden, gefolgt von der Zugabe von 0,5 bis 5,0 Äquivalenten Aminopropyltriethoxysilan zu dieser Mischung, woraufhin die Mischung über einen Zeitraum von 12 bis 72 Stunden bei einer Temperatur von 20°C bis 40°C zur Reaktion gebracht wird.

10. Verfahren zur Gewinnung der Verbindung der Formel I nach Anspruch 9, **dadurch gekennzeichnet, dass** das in Schritt II erhaltene Produkt gereinigt und mit einem geeigneten Lösungsmittel wie Acetonitril oder Methanol gewaschen wird.

11. Verfahren zur Gewinnung der Verbindung der Formel I nach Anspruch 6, **dadurch gekennzeichnet, dass** Schritt III unter Verwendung einer 0,1- bis 2,0-molaren Lösung der geschützten und silanisierten α-Aminosäure, gelöst in Ethanol oder Methanol, bei einer Temperatur von 40 bis 80°C und durch Kombination mit Kaliumcarbonat (oder Essigsäure) in einem Verhältnis von 0,1 bis 3,0 Äquivalenten über einen Zeitraum von 0,1 bis 12 Stunden durchgeführt wird, woraufhin zu dieser Mischung langsam 1,0 bis 20 Äquivalente Siliciumalkoxid und nachfolgend 30 bis 100 Äquivalente destillierten Wassers unter ständigem Rühren der Reaktionsmischung für 0,5 bis 24 Stunden bei 40 bis 80°C hinzugegeben werden.

12. Verfahren zur Gewinnung der Verbindung der Formel I nach Anspruch 11, **dadurch gekennzeichnet, dass** zum Abschluss von Schritt III das Produkt zur Alterung bei einer Temperatur von 20 bis 30°C über einen Zeitraum von 24 bis 72 Stunden belassen wird, woraufhin das Material bei einer Temperatur von 40 bis 90°C für 24 bis 72 Stunden getrocknet wird, bis ein trockener Feststoff erhalten wird.

13. Verfahren zur Gewinnung der Verbindung der Formel I nach Anspruch 11, **dadurch gekennzeichnet, dass** am Ende von Schritt III das Produkt gereinigt und mit Methanol und Dichlormethan gewaschen wird.

14. Verfahren zur Gewinnung der Verbindung der Formel I nach Anspruch 6, **dadurch gekennzeichnet, dass** Schritt IV unter Verwendung von 3 M Salzsäure oder Trifluoressigsäure in Ethylacetat in einem Verhältnis von 0,1 bis 5,0 ml Säure pro 1 g des in Schritt III erhaltenen trockenen Feststoffs durchgeführt wird.

15. Verfahren zur Gewinnung der Verbindung der Formel I, **dadurch gekennzeichnet, dass** es folgende Schritte umfasst: Schützen der Aminogruppe einer α-Aminosäure durch Reaktion mit einer Schutzgruppe wie etwa Boc₂O (SCHRITT I); anorganische Polymerisierung von APTES mit Siliciumalkoxid (SCHRITT V); eine Fixierungsreaktion zwischen der geschützten α-Aminosäure (das Produkt von Schritt I) und dem in Schritt Verhaltenen Feststoff (SCHRITT VI); Entfernen der Schutzgruppe von der Aminogruppe der α-Aminosäure mithilfe von 3 M Salzsäure oder Trifluoressigsäure in Ethylacetat (SCHRITT IV).

16. Verfahren zur Gewinnung der Verbindung der Formel I nach Anspruch 15, **dadurch gekennzeichnet, dass** Schritt V unter Verwendung einer Mischung von in Ethanol oder Methanol gelöstem 0,1 bis 2,0 M APTES bei einer Temperatur von 40 bis 80°C durchgeführt wird, woraufhin zu dieser Mischung langsam 1,0 bis 20 Äquivalente Siliciumalkoxid und nachfolgend 30 bis 100 Äquivalente destillierten Wassers unter ständigem Rühren der Reaktionsmischung für 0,1 bis 12 Stunden bei 40 bis 80°C hinzugegeben werden.

17. Verfahren zur Gewinnung der Verbindung der Formel I nach Anspruch 16, **dadurch gekennzeichnet, dass** zum Abschluss von Schritt V das Produkt zur Alterung bei einer Temperatur von 20 bis 30°C über einen Zeitraum von 24 bis 72 belassen wird, woraufhin das Material bei einer Temperatur von 40 bis 90°C für 24 bis 72 Stunden getrocknet wird, bis ein trockener Feststoff erhalten wird.

18. Verfahren zur Gewinnung der Verbindung der Formel I nach Anspruch 15, **dadurch gekennzeichnet, dass** Schritt VI unter Verwendung einer 0,1- bis 1,5-molaren Lösung der durch Boc geschützten α-Aminosäure in Acetonitril oder Methanol durchgeführt wird, die zwischen 0°C und 40°C in einer Stickstoffatmosphäre gerührt wird und der 0,5 bis 3,0 Äquivalente Kaliumcarbonat und 0,5 bis 3,0 Äquivalente O-(Benzotriazol-1-yl)-N,N,N',N'-tetramethyluronium-tetrafluorborat unter ständigem Rühren über einen Zeitraum von 10 bis 60 Minuten hinzugefügt werden, woraufhin der in Schritt V erhaltene Feststoff zur Reaktionsmischung in einem Masse-Volumen-Verhältnis (g/ml) von 1:5 bis 1:30 zugegeben wird und anschließend 12 bis 72 Stunden lang bei einer Temperatur von 20°C bis 40°C gehalten wird.

19. Verfahren zur Gewinnung der Verbindung der Formel I nach Anspruch 18, **dadurch gekennzeichnet, dass** das in Schritt IV erhaltene Produkt gereinigt und unter Verwendung eines geeigneten Lösungsmittels wie Acetonitril oder Methanol gewaschen wird.

20. Verwendung der Verbindung der Formel I nach einem der Ansprüche 1 bis 5 als Katalysator in Reaktionen zur Bildung von Kohlenstoff-Kohlenstoff-Bindungen chiraler Natur, wie etwa Aldolkondensation, Michael-Addition, Mannich-Reaktion oder Henry-Reaktion.

21. Verwendung nach Anspruch 20, wobei wässrige oder organische Reaktionsmittel bei Temperaturen im Bereich von -78°C bis 40°C eingesetzt werden.

22. Verwendung nach Anspruch 20, wobei wässrige Reaktionsmittel basierend auf einem Phosphatpuffer pH 7 bei Temperaturen im Bereich von 0°C bis 10°C eingesetzt werden.

## Revendications

1. Un composé de formule :
--- [O-Si-C₃H₆-NH-R₁-OCO-(CNH)-R₂-R₂]_{X}-[O-Si]_{Y}--- Formule I
dans lequel R₁ est une chaîne aliphatique -(CH₂)n- avec n étant défini de 0 à 9 et -OCO-(CNH)-R₂-R₂ correspond à l'α-aminoacide utilisé en tant que catalyseur organique ; dans lequel ledit α-aminoacide est n'importe lequel de ceux spécifiés dans le tableau suivant :
| Nom de l'α-aminoacide | Formule chimique |
|---|---|
| Proline | 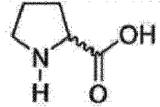 |
| Valine | 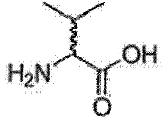 |
| Alanine | 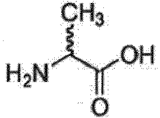 |
| Phénylglycine | 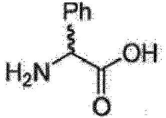 |
| Phénylalanine | 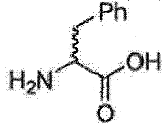 |
| Leucine | 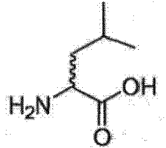 |
| Isoleucine | 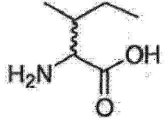 |
| Méthionine | 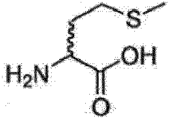 |
; et dans lequel les valeurs X et Y sont conservées dans un rapport allant de 1:1 à 1:20.

2. Le composé de Formule I selon la revendication 1, avec n=0 et -OCO-(CNH)-R₂-R₂. correspondant à de la Proline.

3. Le composé de Formule I selon la revendication 1, avec n=9 et -OCO-(CNH)-R₂-R₂ correspondant à de la Proline.

4. Le composé de Formule I selon la revendication 2, avec les valeurs X=1 et Y=3.

5. Le composé de Formule I selon la revendication 3, avec les valeurs X=1 et Y=6.

6. Un procédé pour obtenir le Composé de Formule I, **caractérisé en ce qu'**il comprend les étapes suivantes : protection du groupe amino d'un α-aminoacide au moyen de la réaction avec un groupe de protection tel que Boc₂O (ÉTAPE I) ; silanisation de la liaison α-aminoacide protégée via le groupe carboxyle au moyen d'aminopropyltriéthoxysilane (ÉTAPE II) ; polymérisation inorganique de l'aminoacide protégé et silanisé au moyen d'un alcoxyde de silicium en présence ou en l'absence d'un catalyseur acide ou basique, en conservant un rapport aminoacide protégé et silanisé:alcoxyde allant de 1:1 à 1:20 (ÉTAPE III) ; retrait de la protection du groupe amino de l'α-aminoacide en utilisant 3M d'acide chlorhydrique ou d'acide trifluoroacétique dans de l'acétate d'éthyle (ÉTAPE IV).

7. Un procédé pour obtenir le Composé de Formule I selon la revendication 6, **caractérisé en ce que** l'étape I est réalisée en utilisant une solution de 0.1 à 2.0 α-aminoacide dans un mélange d'eau distillée et de 1,4 dioxane (dans un rapport de volume allant de 1:0.5 à 1:2) entre 0°C et 30°C, en ajoutant de 0.5 à 3.0 équivalents de carbonate de diterbutyle.

8. Un procédé pour obtenir le Composé de Formule I selon la revendication 7, **caractérisé en ce que** 0.3 à 3.0 équivalents de triéthylamine sont ajoutés et le mélange mis à réagir entre 20°C et 40°C pendant une période s'étendant de 12 à 72 heures.

9. Un procédé pour obtenir le Composé de Formule I selon la revendication 6, **caractérisé en ce que** l'étape II est réalisée en utilisant un mélange d'α-aminoacide protégé dans 0.1 à 2.0M d'acétonitrile ou méthanol, auquel sont ajoutés de 0.5 à 3.0 équivalents de carbonate de potassium et de 0.5 à 3.0 équivalents de O-(benzotriazole-1-yl)-N,N,N',N'-tétraméthyluronium-tétrafluoroborate, à une température allant de 0°C à 40°C dans une atmosphère d'azote pendant une période de 10 à 60 minutes, puis par l'ajout à ce mélange de 0.5 à 5.0 équivalents d'aminopropyltriéthoxysilane, en mettant le mélange à réagir pendant une période de 12 à 72 heures à une température entre 20°C et 40°C.

10. Un procédé pour obtenir le Composé de Formule I selon la revendication 9, **caractérisé en ce que** le produit obtenu dans l'étape II est purifié et lavé en utilisant un solvant adapté tel que l'acétonitrile ou le méthanol.

11. Un procédé pour obtenir le Composé de Formule I selon la revendication 6, **caractérisé en ce que** l'étape III est réalisée en utilisant une solution de 0.1 à 2.0M de l'α-aminoacide protégé et silanisé dissous dans de l'éthanol ou du méthanol à une température entre 40 et 80°C et en mélangeant avec du carbonate de potassium (ou de l'acide acétique) dans un rapport de 0.1 à 3.0 équivalents pendant une période de 0.1 à 12 heures, après laquelle à ce mélange sont lentement ajoutés de 1.0 à 20 équivalents d'alcoxyde de silicium, et plus tard de 30 à 100 équivalents d'eau distillée, en agitant continuellement le mélange de réaction à une température de 40 à 80°C pendant 0.5 à 24 heures.

12. Un procédé pour obtenir le Composé de Formule I selon la revendication 11, **caractérisé en ce que**, pour conclure l'étape III, le produit est mis à vieillir et à reposer à une température de 20 à 30°C pendant une période de 24 à 72 heures, après laquelle la matière est séchée à une température de de 40 à 90°C pendant une période de 24 à 72 heures jusqu'à l'obtention d'un solide sec.

13. Un procédé pour obtenir le Composé de Formule I selon la revendication 11, **caractérisé en ce qu'**à la fin de l'étape III, le produit est purifié et lavé avec du méthanol et du dichlorométhane.

14. Un procédé pour obtenir le Composé de Formule I selon la revendication 6, **caractérisé en ce que** l'étape IV est réalisée en utilisant 3M d'acide chlorhydrique ou d'acide trifluoroacétique dans de l'acétate d'éthyle dans un rapport de 0.1 à 5.0 mL d'acide pour 1 g du solide sec obtenu dans l'étape III.

15. Un procédé pour obtenir le Composé de Formule I, **caractérisé en ce qu'**il comprend les étapes suivantes : protection du groupe amino d'un α-aminoacide au moyen de la réaction avec un groupe de protection tel que Boc₂O (ÉTAPE I) ; polymérisation inorganique d'APTES avec alcoxyde de silicium (ÉTAPE V) ; une réaction de fixation entre de l'α-aminoacide protégé (le produit de l'étape I) et le solide obtenu dans l'étape V (ÉTAPE VI) ; retrait de la protection du groupe amino de l'α-aminoacide au moyen de 3M d'acide chlorhydrique ou d'acide trifluoroacétique dans de l'acétate d'éthyle (ÉTAPE IV).

16. Un procédé pour obtenir le Composé de Formule I selon la revendication 15, **caractérisé en ce que** l'étape V est réalisée en utilisant un mélange d'APTES dissous dans de l'éthanol ou du méthanol de 0.1 à 2.0M à une température de 40 à 80°C, après quoi à ce mélange sont lentement ajoutés de 1.0 à 20 équivalents d'alcoxyde de silicium et plus tard de 30 à 100 équivalents d'eau distillée, en agitant continuellement le mélange de réaction à une température de 40 à 80°C pendant 0.1 à 12 heures.

17. Un procédé pour obtenir le Composé de Formule I selon la revendication 16, **caractérisé en ce que**, pour terminer l'étape V, le produit est mis à vieillir et à reposer à une température de 20 à 30°C pendant une période de 24 à 72 heures, après laquelle la matière est séchée à une température de de 40 à 90°C pendant une période de 24 à 72 heures jusqu'à l'obtention d'un solide sec.

18. Un procédé pour obtenir le Composé de Formule I selon la revendication 15, **caractérisé en ce que** l'étape VI est réalisée en utilisant une solution de 0.1 à 1.5M de l'α-aminoacide protégé par Boc dans de l'acétonitrile ou du méthanol agitée à une température allant de 0°C à 40°C dans une atmosphère d'azote, à laquelle sont ajoutés de 0.5 à 3.0 équivalents de carbonate de potassium et de 0.5 à 3.0 équivalents de O-(benzotriazole-1-yl)-N,N,N',N'-tétraméthyluronium-tétrafluoroborate, sous agitation continue pendant une période de 10 à 60 minutes, après laquelle au mélange de réaction est ajouté le solide obtenu dans l'étape V dans un rapport masse/volume (g/mL) allant de 1/5 à 1/30, en conservant ensuite le mélange de réaction à une température de 20°C à 40°C pendant une période de 12 à 72 heures.

19. Un procédé pour obtenir le Composé de Formule I selon la revendication 18, **caractérisé en ce que** le produit obtenu dans l'étape IV est purifié et lavé en utilisant un solvant adapté tel que l'acétonitrile ou le méthanol.

20. Utilisation du Composé de Formule I selon n'importe laquelle des revendications 1 à 5, en tant que catalyseur dans des réactions de formation de liaisons carbone-carbone de nature chirale, telles que condensation aldolique, addition de Michael, réaction de Mannich, ou réaction de Henry.

21. Utilisation selon la revendication 20, en utilisant des moyens de réaction aqueux ou organiques dans la gamme de températures allant de -78°C à 40°C.

22. Utilisation selon la revendication 20, en utilisant des moyens de réaction aqueux avec une base de tampon phosphate pH 7 dans une gamme de températures allant de 0°C à 10°C.
